# EUROPEAN PATENT SPECIFICATION

(11) **EP 3 185 902 B1**
(45) Date of publication and mention of the grant of the patent: **29.07.2020**
(21) Application number: 15835936.4
(22) Date of filing: 26.08.2015
(51) Int. Cl.: A61K 39/00, A61K 45/00, A61P 17/06, C07K 16/28

(54) **METHOD FOR TREATING PSORIASIS PATIENT WHICH RECEIVED ANTI-TNF-ALPHA ANTIBODY THERAPY**
VERFAHREN ZUR BEHANDLUNG EINES PSORIASISPATIENTEN, DER EINE ANTI-TNF-ALPHA-ANTIKÖRPERTHERAPIE ERHALTEN HAT
PROCÉDÉ POUR TRAITER UN PATIENT SOUFFRANT DE PSORIASIS QUI A REÇU UN TRAITEMENT PAR ANTICORPS ANTI-TNF-ALPHA

(30) Priority: 26.08.2014 US 201462041862 P
(43) Date of publication of application: 05.07.2017
(73) Proprietor: Amgen K-A, Inc., Thousand Oaks, CA 91320-1799 (US)
(72) Inventor: NAKAGAWA, Hidemi, Tokyo 105-8471 (JP); MATSUDO, Hiroki, Tokyo 100-8185 (JP)
(74) Representative: Carpmaels & Ransford LLP
(86) International application number: PCT/JP2015/004306
(87) International publication number: WO 2016/031250

(56) References cited:
- WO-A1-2011/088120
- WO-A1-2012/045848
- WO-A2-2008/054603
- Anonymous: "NCT02052609 on 2014_01_31: ClinicalTrials.gov Archive", , 31 January 2014 (2014-01-31), XP055189143, Retrieved from the Internet: URL:https://clinicaltrials.gov/archive/NCT 02052609/2014_01_31 [retrieved on 2015-05-13]
- Y. TERAKI ET AL: "A case of generalized psoriasiform and pustular eruption induced by infliximab: evidence for skin-homing Th17 in the pathogenesis : Correspondence", BRITISH JOURNAL OF DERMATOLOGY, vol. 163, no. 6, 4 November 2010 (2010-11-04), pages 1347-1351, XP055445850, UK ISSN: 0007-0963, DOI: 10.1111/j.1365-2133.2010.10002.x
- K. YAMASAKI ET AL: "Efficacy and safety of brodalumab in patients with generalized pustular psoriasis and psoriatic erythroderma: results from a 52-week, open-label study", BRITISH JOURNAL OF DERMATOLOGY, vol. 176, no. 3, 2 October 2016 (2016-10-02), pages 741-751, XP055445793, UK ISSN: 0007-0963, DOI: 10.1111/bjd.14702
- FRANCISCO KERDEL ET AL: "An evolution in switching therapy for psoriasis patients who fail to meet treatment goals : Switching therapy in psoriasis", DERMATOLOGIC THERAPY, vol. 28, no. 6, 1 November 2015 (2015-11-01), pages 390-403, XP055376490, DK ISSN: 1396-0296, DOI: 10.1111/dth.12267
- ANDREA PRIMIANI MOY ET AL: "Immunologic Overlap of Helper T-Cell Subtypes 17 and 22 in Erythrodermic Psoriasis and Atopic Dermatitis", JAMA DERMATOLOGY, vol. 151, no. 7, 1 July 2015 (2015-07-01), page 753, XP055445853, US ISSN: 2168-6068, DOI: 10.1001/jamadermatol.2015.2
- BENJAMIN FARAHNIK ET AL: "Brodalumab for the Treatment of Psoriasis: A Review of Phase III Trials", DERMATOLOGY AND THERAPY, vol. 6, no. 2, 25 May 2016 (2016-05-25), pages 111-124, XP055445787, ISSN: 2193-8210, DOI: 10.1007/s13555-016-0121-x
- P.J.MEASE ET AL.: 'Managing patients with psoriatic disease: the diagnosis and pharmacologic treatment of psoriatic arthritis in patients with psoriasis.' DRUGS vol. 74, no. 4, 01 March 2014, pages 423 - 441, XP055411681 DOI: 10.1007/S40265-014-0191-Y

### SEQUENCE LISTING

<110> Kirin-Amgen, Inc.
<120> Method for treating psoriasis patient which received anti-TNF-alpha
   antibody therapy
<130> W518973
<150> US 62/041,862
   <151> 2014-08-26
<160> 8
<170> PatentIn version 3.3
<210> 1
   <211> 5
   <212> PRT
   <213> Artificial
<220>
   <223> Description of artificial sequence; amino acid sequence of AM-14_H_CDR1
<400> 1
   Arg Tyr Gly Ile Ser
   1               5
<210> 2
   <211> 17
   <212> PRT
   <213> Artificial
<220>
   <223> Description of artificial sequence; amino acid sequence of AM-14_H_CDR2
<400> 2
   Trp Ile Ser Thr Tyr Ser Gly Asn Thr Asn Tyr Ala Gln Lys Leu Gln
   1               5                   10                  15
   Gly
<210> 3
   <211> 7
   <212> PRT
   <213> Artificial
<220>
   <223> Description of artificial sequence; amino acid sequence of AM-14_H_CDR3
<400> 3
   Arg Gln Leu Tyr Phe Asp Tyr
   1               5
<210> 4
   <211> 11
   <212> PRT
   <213> Artificial
<220>
   <223> Description of artificial sequence; amino acid sequence of AM-14_L_CDR1
<400> 4
   Arg Ala Ser Gln Ser Val Ser Ser Asn Leu Ala
   1 5 10
<210> 5
   <211> 7
   <212> PRT
   <213> Artificial
<220>
   <223> Description of artificial sequence; amino acid sequence of AM-14_L_CDR2
<400> 5
   Asp Ala Ser Thr Arg Ala Thr
   1 5
<210> 6
   <211> 9
   <212> PRT
   <213> Artificial
<220>
   <223> Description of artificial sequence; amino acid sequence of AM-14_L_CDR3
<400> 6
   Gln Gln Tyr Asp Asn Trp Pro Leu Thr
   1 5
<210> 7
   <211> 116
   <212> PRT
   <213> Artificial
<220>
   <223> Description of artificial sequence; amino acid sequence of AM-14_VH
<400> 7
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Arg Tyr
                   20                  25                  30
   Gly Ile Ser Trp Val Arg Gln Ala Pro Gly Gln Gly Leu Glu Trp Met
           35                  40                  45
   Gly Trp Ile Ser Thr Tyr Ser Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Arg Gln Leu Tyr Phe Asp Tyr Trp Gly Gln Gly Thr Leu Val
               100                 105                 110
   Thr Val Ser Ser
           115
<210> 8
   <211> 107
   <212> PRT
   <213> Artificial
<220>
   <223> Description of artificial sequence; amino acid sequence of AM-14_VL
<400> 8
   Glu Ile Val Met Thr Gln Ser Pro Ala Thr Leu Ser Val Ser Pro Gly
   1               5                   10                  15
   Glu Arg Ala Thr Leu Ser Cys Arg Ala Ser Gln Ser Val Ser Ser Asn
               20                  25                  30
   Leu Ala Trp Phe Gln Gln Lys Pro Gly Gln Ala Pro Arg Pro Leu Ile
           35                  40                  45
   Tyr Asp Ala Ser Thr Arg Ala Thr Gly Val Pro Ala Arg Phe Ser Gly
       50                  55                  60
   Ser Gly Ser Gly Thr Asp Phe Thr Leu Thr Ile Ser Ser Leu Gln Ser
   65                  70                  75                      80
   Glu Asp Phe Ala Val Tyr Tyr Cys Gln Gln Tyr Asp Asn Trp Pro Leu
                   85                  90                  95
   Thr Phe Gly Gly Gly Thr Lys Val Glu Ile Lys
                       100                 105

## Description

### Technical Field

The present invention relates to a therapeutic agent for psoriasis that is administered to a psoriasis patient that cannot be treated with an anti-TNF-alpha antibody, comprising an IL- 17RA antagonist as an active ingredient. The present invention also relates to a method for the treatment of psoriasis, comprising administering an IL-17RA antagonist to a psoriasis patient that cannot be treated with an anti-TNF-alpha antibody.

### Background Art

Overproduction of cytokines contributes to the development of a number of inflammatory autoimmune diseases including psoriasis, psoriatic arthritis, and asthma. The interleukin (IL)-17 family of cytokines is comprised of 6 family members with the following nomenclature: IL-17A, IL-17B, IL-17C, IL-17D, IL-25 (also known as IL-17E), and IL-17F. Interleukin-17A, IL-17F, and IL-17A/F are hallmark proinflammatory cytokines produced by T helper cells producing IL-17 (Th17) cells and innate immune cells that have been shown to contribute to an inflammatory response in models of autoimmune disorders (NPLs 1-6).

Interleukin-17A, IL-17F, and IL-17A/F have pleiotropic activities, including the induction of proinflammatory mediators from epithelial cells, endothelial cells and fibroblasts that promote tissue inflammation and destruction; the proliferation, maturation, and chemotaxis of neutrophils; and the maturation of dendritic cells (NPLs 7, 8).

Interleukin-25 is produced by epithelial cells, T-helper 2 (Th2) cells, eosinophils, and basophils and is more often associated with Th2-type inflammatory pathologies such as asthma (NPLs 9-11). Interleukin-17C is also produced by epithelial cells and the biological activities of this cytokine are being explored but appear to be similar to the activities of IL-17A and IL-17F (NPL 12).

Interleukin-17RA is a type I transmembrane receptor that is found on a wide variety of cell types including but not limited to fibroblasts, epithelial cells, and monocytes (NPL 13). Interleukin-17A, IL-17F, IL-17A/F, IL-17C, and IL-25 stimulate cellular responses by binding to IL-17RA. Interleukin-17A, IL-17F, and IL-17A/F signal via a heteromeric IL-17RA/IL-17RC complex, IL-17C signals via a heteromeric IL 17RA/IL-17RE complex, and IL-25 signals via a heteromeric IL-17RA/IL-17RB complex (NPLs 12, 14-17). Interleukin-17RA blockade represents a novel mechanism to inhibit the inflammation and clinical symptoms associated with autoimmune and inflammatory diseases including but not limited to psoriasis, psoriatic arthritis, and asthma.

AM-14 is a human CHO cell-derived IgG2 monoclonal antibody that binds to human IL-17RA and blocks the biological activities of IL-17A, IL-17F, IL-17A/F heterodimer, and IL-25 involved in the various autoimmune disorder and inflammatory disorder (NPLs 18-20). NPL21 describes an extension study of KHK4827 in subjects with plaque psoriasis (psoriasis vulgaris, psoriatic arthritis), pustular psoriasis (generalized) and psoriatic erythroderma. NPL22 describes managing patients with psoriatic disease. NPL23 describes a case of generalized psoriasiform and pustular eruption induced by infliximab. NPL24 describes evolution in switching therapy for psoriasis patients who fail to meet treatment goals.

### Citation List

### Non Patent Literature

NPL 1: Nat Rev Immunol. 2010;10:479-490
NPL 2: Cell. 2010; 140:845-858
NPL 3: Nature Rev Immunol.2009;9(8):556-567
NPL 4: Immunity. 2008;28(4):454-67
NPL 5: J Exp Med. 2005;201(2):233-240
NPL 6: Nat Immunol. 2005;6(11):1133-1141
NPL 7: Immunity.2004;21:467-476
NPL 8: J Immunol. 1999;162(1):577-584
NPL 9: Immunological Rev. 2008;226:172-190
NPL 10: J Exp Med. 2007;204(8):1837-1847
NPL 11: Immunity. 2001;15:985-995
NPL 12: Nat Immunol.2011;12(12):1159-1166
NPL 13: Cytokine. 1997;9(11):794-800
NPL 14: Nat Immunol. 2011; 12(12):1151-1158
NPL 15: J Immunol. 2008;181(6):4299-4310
NPL 16: J Immunol. 2008;181(4):2799-2805
NPL 17: J Immunol. 2006;177(1):36-39
NPL 18: Immunity. 2008;28(4):454-67
NPL 19: J. Allergy. Clin. Immunol. 2007;120(6):1324-31
NPL 20: J Immunol. 2005;175(1):404-12
NPL 21: NCT02052609 on 2014_01_31: ClinicalTrials.gov Archive
NPL 22: Drugs 74(4):423-441
NPL 23: British Journal of Dermatology 163(6): 1347-1352
NPL 24: Dermatologic Therapy 28(6):390-403

It is an object of the present invention to provide therapeutic agent for psoriasis that is administered to a psoriasis patient that cannot be treated with an anti-TNF-alpha antibody, comprising an IL-17RA antagonist as an active ingredient. The present invention is also intended to provide an IL-17RA antagonist for use in a method for the treatment of psoriasis, comprising administering an IL-17RA antagonist to a psoriasis patient that cannot be treated with an anti-TNF-alpha antibody.

### Summary of the invention

The invention provides an IL-17RA antagonist for use in a method for the treatment of psoriasis, wherein the method comprises administering an IL-17RA antagonist to a psoriasis patient that cannot be treated with an anti-TNF-alpha antibody,
wherein the patient that cannot be treated with the anti-TNF-alpha antibody is:
   (a) a patient that is ineffective to the anti-TNF-alpha antibody, wherein ineffective means a clinical global impression (CGI) of 3 or 4, or
   (b) a patient that is of insufficient tolerability to the anti-TNF-alpha antibody,
wherein insufficient tolerability means severe side effects to a degree at
which a patient cannot tolerate occur,
   wherein the IL-17RA antagonist is an anti-IL-17RA antibody or an antibody fragment thereof, and wherein the anti-IL-17RA antibody is a monoclonal antibody in which CDR1, CDR2, and CDR3 of a heavy chain variable domain (VH) of the antibody comprise the amino acid sequences shown in SEQ ID NOs:1, 2, and 3, respectively, and CDR1, CDR2, and CDR3 of a light chain variable domain (VL) of the antibody comprise the amino acid sequences shown in SEQ ID NOs:4, 5, and 6, respectively,
and wherein the anti-TNF-alpha antibody is at least one selected from adalimumab, infliximab, certolizumab pegol, certolizumab, and golimumab.

### Summary of the disclosure

The present disclosure relates to the following (1) to (34).
(1) A therapeutic agent for pustular psoriasis or psoriatic erythroderma that is administered to a psoriasis patient that has been administered with an anti-TNF-alpha antibody, comprising an IL-17RA antagonist as an active ingredient.
(2) The therapeutic agent described in (1), wherein the anti-TNF-alpha antibody is at least one antibody selected from adalimumab, infliximab, certolizumab pegol, certolizumab, and golimumab.
(3) The therapeutic agent described in (1) or (2), wherein the IL-17RA antagonist is an anti-IL-17RA antibody or an antibody fragment thereof.
(4) The therapeutic agent described in (3), wherein the anti-IL-17RA antibody is selected from the following a) and b):
   a) a monoclonal antibody in which a complementarity determining region (hereinafter, referred to as CDR)1, CDR2, and CDR3 of a heavy chain variable region (hereinafter, referred to as VH) of the antibody comprise the amino acid sequences shown in SEQ ID NOs:1, 2, and 3, respectively, and CDR1, CDR2, and CDR3 of a light chain variable region (hereinafter, referred to as VL) of the antibody comprise the amino acid sequences shown in SEQ ID NOs:4, 5, and 6, respectively; and
   b) a monoclonal antibody in which VH of the antibody comprises the amino acid sequence shown in SEQ ID NO:7, and VL of the antibody comprises the amino acid sequence shown in SEQ ID NO:8.
(5) The therapeutic agent described in any one of (1) to (4), wherein the clinical global impression (hereinafter, also referred to as CGI) of a patient after administration of the therapeutic agent becomes 2 or 1.
(6) The therapeutic agent described in any one of (1) to (5), wherein psoriasis area and severity index (hereinafter, referred to as PASI) score of a patient after administration of the therapeutic agent is lower than that before administration of the therapeutic agent.
(7) A therapeutic agent for psoriasis that is administered to a psoriasis patient that cannot be treated with an anti-TNF-alpha antibody, comprising an IL-17RA antagonist as an active ingredient.
(8) The therapeutic agent described in (7), wherein the patient that cannot be treated with the anti-TNF-alpha antibody is a patient that does not respond to the anti-TNF-alpha antibody or that is of insufficient tolerability to the anti-TNF-alpha antibody.
(9) The therapeutic agent described in (7) or (8), wherein the anti-TNF-alpha antibody is at least one selected from adalimumab, infliximab, certolizumab pegol, certolizumab, and golimumab.
(10) The therapeutic agent described in any one of (7) to (9), wherein the IL-17RA antagonist is an anti-IL-17RA antibody or an antibody fragment thereof.
(11) The therapeutic agent described in (10), wherein the anti-IL-17RA antibody is selected from the following a) and b):
   a) a monoclonal antibody in which CDR1, CDR2, and CDR3 of VH of the antibody comprise the amino acid sequences shown in SEQ ID NOs:1, 2, and 3, respectively, and CDR1, CDR2, and CDR3 of VL of the antibody comprise the amino acid sequences shown in SEQ ID NOs:4, 5, and 6, respectively; and
   b) a monoclonal antibody in which VH of the antibody comprises the amino acid sequence shown in SEQ ID NO:7, and VL of the antibody comprises the amino acid sequence shown in SEQ ID NO:8.
(12) The therapeutic agent described in any one of (7) to (11), wherein the psoriasis is at least one psoriasis selected from psoriasis vulgaris, psoriasis arthropica, pustular psoriasis, psoriatic erythroderma, and psoriasis guttata.
(13) The therapeutic agent described in any one of (7) to (12), wherein the CGI of a patient after administration of the therapeutic agent becomes 2 or 1.
(14) The therapeutic agent described in any one of (7) to (13), wherein the PASI score of a patient after administration of the therapeutic agent is lower than that before administration of the therapeutic agent.
(15) A method for the treatment of pustular psoriasis or psoriatic erythroderma, comprising administering an IL-17RA antagonist to a psoriasis patient that has been administered with an anti-TNF-alpha antibody.
(16) The method described in (15), wherein the anti-TNF-alpha antibody is at least one selected from adalimumab, infliximab, certolizumab pegol, certolizumab, and golimumab.
(17) The method described in (15) or (16), wherein the IL-17RA antagonist is an anti-IL-17RA antibody or an antibody fragment thereof.
(18) The method described in (17), wherein the anti-IL-17RA antibody is selected from the following a) and b):
   a) a monoclonal antibody in which CDR1, CDR2, and CDR3 of VH of the antibody comprise the amino acid sequences shown in SEQ ID NOs:1, 2, and 3, respectively, and CDR1, CDR2, and CDR3 of VL of the antibody comprise the amino acid sequences shown in SEQ ID NOs:4, 5, and 6, respectively; and
   b) a monoclonal antibody in which VH of the antibody comprises the amino acid sequence shown in SEQ ID NO:7, and VL of the antibody comprises the amino acid sequence shown in SEQ ID NO:8.
(19) The method described in any one of (15) to (18), wherein the CGI of the patient after the administration of the IL-17RA antagonist becomes 2 or 1.
(20) The method described in any one of (15) to (19), wherein the PASI score of a patient after the administration of the IL-17RA antagonist is lower than that before the administration of the antagonist.
(21) The method described in any one of (15) to (20), wherein the IL-17RA antagonist is administered in a dose of 140 mg or more.
(22) The method described in any one of (15) to (21), wherein the IL-17RA antagonist is administered in a dose of 140 mg or 210 mg.
(23) The method described in any one of (15) to (22), wherein the IL-17RA antagonist is administered in a dose of 140 mg or 210 mg on the 1^{st} day, the 1^{st} week and the 2^{nd} week, and thereafter once every two weeks.
(24) A method for the treatment of psoriasis, comprising administering an IL-17RA antagonist to a psoriasis patient that cannot be treated with an anti-TNF-alpha antibody.
(25) The method described in (24), wherein the patient that cannot be treated with the anti-TNF-alpha antibody is a patient that is ineffective to the anti-TNF-alpha antibody or that is of insufficient tolerability to the anti-TNF-alpha antibody.
(26) The method described in (24) or (25), wherein the anti-TNF-alpha antibody is at least one selected from adalimumab, infliximab, certolizumab pegol, certolizumab, and golimumab.
(27) The method described in any one of (24) to (26), wherein the IL-17RA antagonist is an anti-IL-17RA antibody or an antibody fragment thereof.
(28) The method described in (27), wherein the anti-IL-17RA antibody is selected from the following a) and b):
   a) a monoclonal antibody in which CDR1, CDR2, and CDR3 of VH of the antibody comprise the amino acid sequences shown in SEQ ID NOs:1, 2, and 3, respectively, and CDR1, CDR2, and CDR3 of VL of the antibody comprise the amino acid sequences shown in SEQ ID NOs:4, 5, and 6, respectively; and
   b) a monoclonal antibody in which VH of the antibody comprises the amino acid sequence shown in SEQ ID NO:7, and VL of the antibody comprises the amino acid sequence shown in SEQ ID NO:8.
(29) The method described in any one of (24) to (28), wherein the psoriasis is at least one selected from psoriasis vulgaris, psoriasis arthropica, pustular psoriasis, psoriatic erythroderma, and psoriasis guttata.
(30) The method described in any one of (24) to (29), wherein the CGI of the patient after the administration of the IL-17RA antagonist becomes 2 or 1.
(31) The method described in any one of (24) to (30), wherein the PASI score of a patient after the administration of the IL-17RA antagonist is lower than that before the administration of the antagonist.
(32) The method described in any one of (24) to (31), wherein the IL-17RA antagonist is administered in a dose of 140 mg or more.
(33) The method described in any one of (24) to (32), wherein the IL-17RA antagonist is administered in a dose of 140 mg or 210 mg.
(34) The method described in any one of (24) to (33), wherein the IL-17RA antagonist is administered in a dose of 140 mg or 210 mg on the 1^{st} day, the 1^{st} week and the 2^{nd} week, and thereafter once every two weeks.

Herein disclosed is a therapeutic agent for pustular psoriasis or psoriatic erythroderma that is administered to a psoriasis patient that has been ad- ministered with an anti-TNF-alpha antibody, comprising an IL-17RA antagonist as an active ingredient; and a therapeutic agent for psoriasis that is administered to a psoriasis patient that cannot be treated with an anti-TNF-alpha antibody, comprising an IL-17RA antagonist as an active ingredient. Herein also disclosed is a method for the treatment of pustular psoriasis or psoriatic erythroderma, comprising administering an IL-17RA antagonist to a psoriasis patient that has been administered with an anti-TNF-alpha antibody; and a method for the treatment of psoriasis, comprising administering an IL-17RA antagonist to a psoriasis patient that cannot be treated with an anti-TNF-alpha antibody.

### Brief Description of Drawings

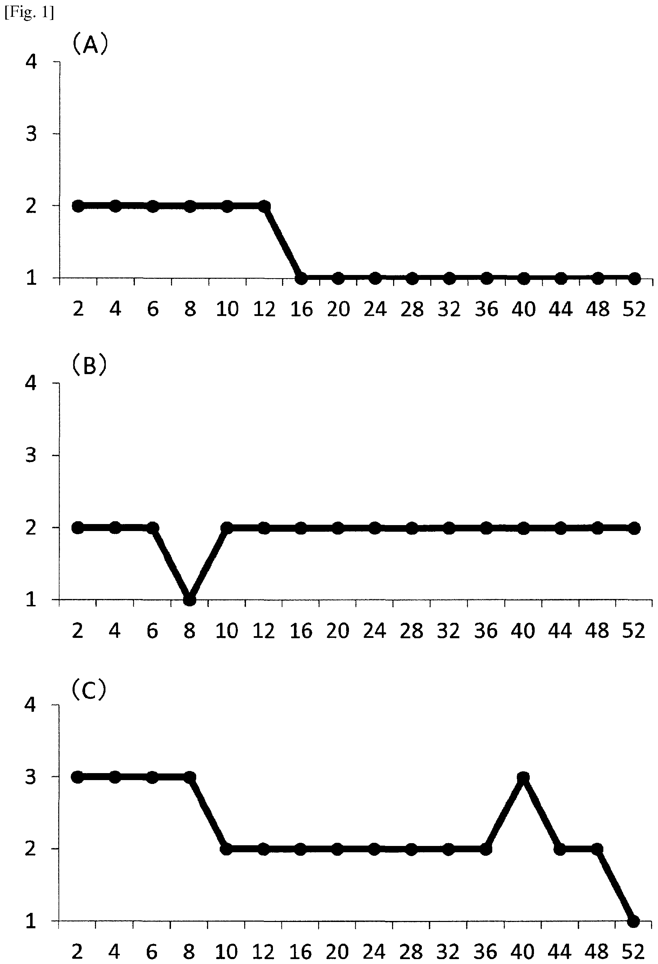

Figs. 1(A) to 1(C) show the therapeutic effect of AM-14 for pustular psoriasis patients that have been administered with anti-TNF-alpha antibodies. The vertical axis represents clinical global impression, and the horizontal axis represents a period after the first AM-14 administration (week). (A) to (C) are results for subjects A to C, respectively.

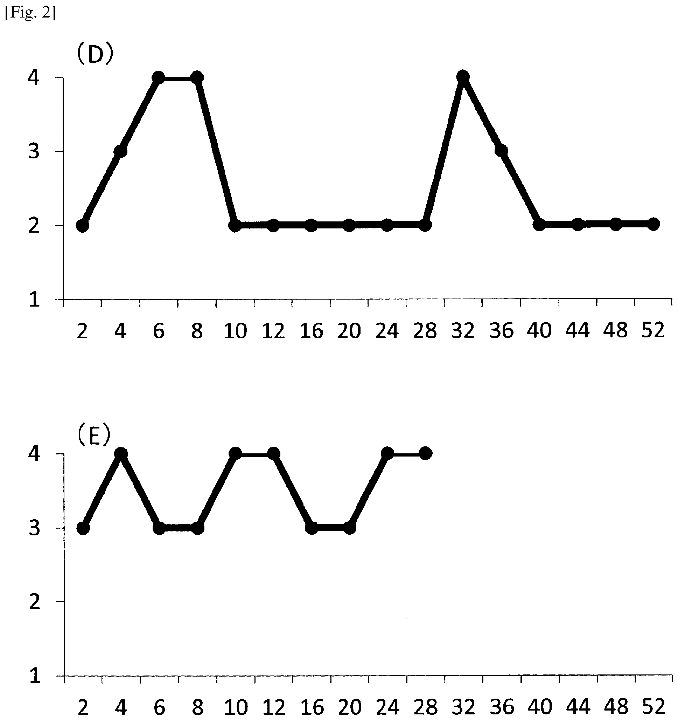

Figs. 2(D) and 2(E) show the therapeutic effect of AM-14 for pustular psoriasis patients that have been administered with anti-TNF-alpha antibodies. The vertical axis represents clinical global impression, and the horizontal axis represents a period after the first AM-14 administration (week). (D) and (E) are results for subjects D and E, respectively.

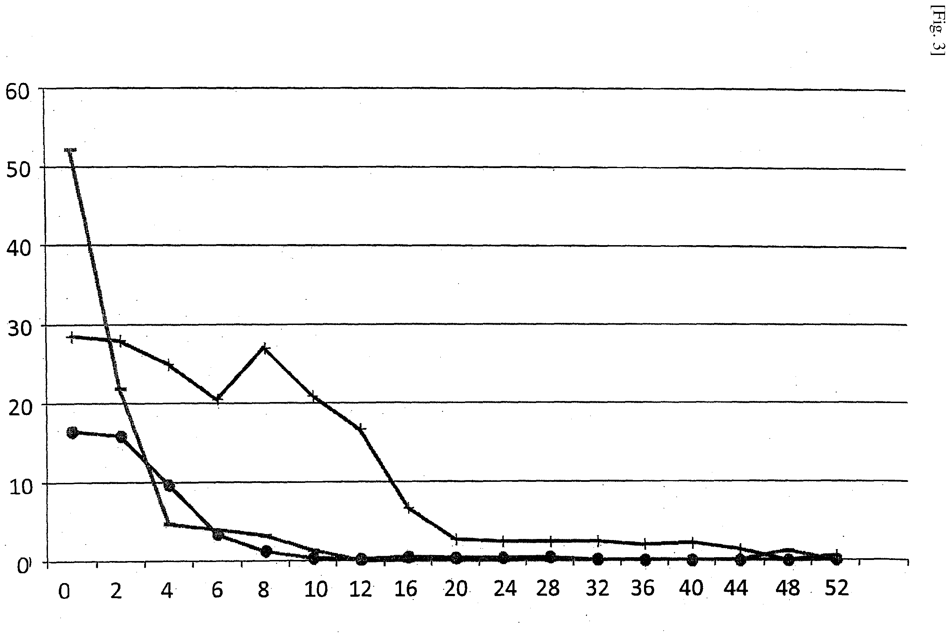

Fig. 3 shows the therapeutic effect of AM-14 for psoriatic erythroderma patients that have been administered with anti-TNF-alpha antibodies. The vertical axis represents PASI score, and the horizontal axis represents a period (week) after the first AM-14 administration. Week 0 of administration is also denoted as the 1st day of administration. The results for subjects F, G, and H are plotted in solid lines with the black circle, plus, and minus symbols, respectively.

### Description of Embodiments

The present disclosure relates to a therapeutic agent for pustular psoriasis or psoriatic erythroderma that is administered to a psoriasis patient that has been administered with an anti-tumor necrosis factor-alpha (hereinafter, referred to as "TNF-alpha") antibody, comprising an IL-17RA antagonist as an active ingredient; and to a therapeutic agent for psoriasis that is administered to a psoriasis patient that cannot be treated with an anti-TNF-alpha, comprising an IL-17RA antagonist as an active ingredient.

The present disclosure also relates to a method for the treatment of pustular psoriasis or psoriatic erythroderma, comprising administering an IL-17RA antagonist to a psoriasis patient that has been administered with an anti-TNF-alpha antibody; and to a method for the treatment of psoriasis, comprising administering an IL-17RA antagonist to a psoriasis patient that cannot be treated with an anti-TNF-alpha antibody.

The anti-TNF-alpha antibody is not particularly limited in the present disclosure, as long as it is an antibody against TNF-alpha. The anti-TNF-alpha antibody is preferably an antibody against human TNF-alpha. Specific examples of the anti-human TNF-alpha antibody include infliximab, adalimumab, certolizumab pegol, certolizumab, golimumab, and the like.

Psoriasis is a chronic skin disease, and in clinical practice, psoriasis is classified into five types, that is, psoriasis vulgaris, psoriasis arthropica, pustular psoriasis, psoriatic erythroderma, and psoriasis guttata, according to the symptom and the expression site. Psoriasis vulgaris is characterized by skin erythema with thickening of the skin, clear boundaries (plaque), and scales. Psoriasis arthropica is a chronic inflammatory disease of disordering joints, tendon sheaths, tendon attachment portions, and an axial skeleton, in addition to the skin or the like. Pustular psoriasis is characterized by forming skin rash called a pustule on erythema. Psoriatic erythroderma is characterized by skin rash over the whole body, and being accompanied with diffuse blush or scales. Psoriasis guttata is characterized by existence of small size of psoriasis rash having a diameter of 1 cm on trunk, and legs and arms.

Examples of the psoriasis concerned with the present invention include psoriasis vulgaris, psoriasis arthropica (also called psoriatic arthropathy), pustular psoriasis, psoriatic erythroderma, psoriasis guttata, and the like. Preferred are psoriasis vulgaris, psoriasis arthropica, pustular psoriasis, and psoriatic erythroderma. More preferred are moderate to severe psoriasis vulgaris and psoriasis arthropica, and pustular psoriasis and psoriatic erythroderma. Further preferred are pustular psoriasis and psoriatic erythroderma. The psoriasis concerned with the present invention includes one that is complicated by plural types of psoriasis described above, and also includes nail psoriasis.

The psoriasis may be of a form involving plaque psoriasis, and is preferably a form involving moderate to severe plaque psoriasis. The psoriasis may be localized or may affect the whole body. Specific examples of localized psoriasis include scalp psoriasis and the like. The psoriasis involving plaque psoriasis mainly refers to psoriasis vulgaris involving plaque psoriasis, and also includes psoriasis arthropica involving joint symptoms in addition to plaque psoriasis.

The pustular psoriasis may be generalized type pustular psoriasis in which pustule appears extensively or localized type pustular psoriasis in which pustule appears in small range, and is preferably generalized type pustular psoriasis. Specific examples of generalized type pustular psoriasis include acute generalized pustular psoriasis, generalized pustular psoriasis, annular and circulate pustular psoriasis, impetigo herpetiformis, afebrile impetigo herpetiformis, juvenile and infantile pustular psoriasis, and the like. Specific examples of localized type pustular psoriasis include pal-moplantar pustulosis, acrodermatitis continua, transient localized pustulization of psoriasis vulgaris, and the like.

The therapeutic agent of the present disclosure means a drug that can lower the degree of severity of pustular psoriasis or psoriatic erythroderma in a psoriasis patient that has been administered with an anti-TNF-alpha antibody. The therapeutic agent of the present disclosure also means a therapeutic agent that can lower the degree of severity of psoriasis in a psoriasis patient that cannot be treated with an anti-TNF-alpha antibody.

The therapeutic method of the present disclosure means a method that can lower the degree of severity of pustular psoriasis or psoriatic erythroderma in a psoriasis patient that has been administered with an anti-TNF-alpha antibody. The method of the present disclosure also means a therapeutic method that can lower the degree of severity of psoriasis in a psoriasis patient that cannot be treated with an anti-TNF-alpha antibody.

In the present disclosure, with regard to the term "that has been administered with an anti-TNF-alpha antibody", it is not particularly limited as to when the anti-TNF-alpha antibody is administered, as long as it is administered to a patient before the therapeutic agent or the IL-17RA antagonist in the therapeutic method is first administered to the patient. After the anti-TNF-alpha antibody is administered, another drug may be administered before the therapeutic agent or the IL-17RA antagonist is first administered.

Examples of the psoriasis patient that cannot be treated with an anti-TNF-alpha antibody include a patient that is ineffective to the anti-TNF-alpha antibody (primary failure or secondary failure) or that is of insufficient tolerability when the anti-TNF-alpha antibody is administered to a psoriasis patient.

As used herein, "primary failure" means that the drug efficacy is insufficient from the first administration, and "secondary failure" means that the efficacy of an administered drug attenuates over the course of the continuous administration. In addition, "insufficient tolerability" means that severe side effects to a degree at which a patient cannot tolerate occurs by the administered drug.

Any improvement of psoriasis symptoms may be determined by using known methods. For example, improvement can be determined by a reduction in the scores of various indices after the administration of the IL-17RA antagonist for use in the therapeutic method of the present invention relative to before the administration, specifically by a reduction in the score of Psoriasis Area and Severity Index (hereinafter, referred to as "PASI"), Nail Psoriasis Severity Index (hereinafter, referred to as "NAPSI"), Psoriasis Scalp Severity Index (hereinafter, referred to as "PSSI"), or static Physician Global Assessments (hereinafter, referred to as "sPGA"); the improvement of Quality of life (hereinafter, referred to as QOL) of a psoriasis patient; and the like.

PASI indicates a degree of severity of skin symptoms of psoriasis, and it can be measured according to the method described in [http://www.kyowa-kirin.co.jp/kayumi/kansen/jinjo_08.html]. NAPSI indicates a degree of severity of symptoms of nail psoriasis, and it can be measured according to the method described in Rich et al. (J. Am. Acad. Dermatol. vol. 49, number 2, p. 206-212 (2003)). PSSI indicates a degree of severity of scalp psoriasis, and it can be measured according to the method described in Leonardi et al. (J. Engl. J. Med. 2012; 366: 1190-9). sPGA indicates a degree of severity of skin symptoms comprehensively evaluated by a physician, and it can be measured according to the method described in H. N. Viswanathan et al. (Journal of Dermatological Treatment Posted online on July 31, 2014. (doi: 10.3109/09546634.2014.943687)). QOL uses DLQI as an indicator, and it can be measured according to the method described in H. N. Viswanathan et al. (Journal of Dermatological Treatment Posted online on July 31, 2014. (doi: 10.3109/09546634.2014.943687)).

Effects of the IL-17RA antagonist for use of the present invention is evaluated by the clinical global impression (hereinafter, referred to as CGI) which determines the change in skin symptoms of psoriasis from the first administration of the antagonist as one of four stages [1: remission, 2: improvement, 3: unchanged, and 4: aggravation], and as a result, the effects can also be confirmed from the fact that CGI becomes 1 (remission) or 2 (improvement).

Improvement of pustular psoriasis symptoms can also be confirmed from the fact that the degree score is reduced after the administration of the antagonist relative to before the administration, or the like, in addition to the method described above. The degree score is a score obtained by scoring the evaluation of skin symptoms (erythema, pustule, edema) of a patient and laboratory findings (fever, leukocyte count, serum CRP level, and serum albumin level) by being accompanied with systemic inflammation, and the degree score can be calculated based on the criteria of the degree of severity of pustular psoriasis described in "Clinical Practice Guideline 2010 for Pustular Psoriasis (generalized type): therapeutic strategy including incorporation of TNF-alpha inhibitor (Iwatsuki Keiji et al.)".

Specifically, examples of an improvement of psoriatic erythroderma symptoms include a reduction of PASI score after the administration of the therapeutic agent or the antagonist relative to before the administration, and the like. More specifically, an improvement of psoriatic erythroderma symptoms means a 10%, 20%, 30%, 40%, 50%, 60%, 70%, 80%, 90%, or 100% reduction, preferably a 100% reduction of PASI score after the administration of the therapeutic agent relative to before the administration.

The term administration as used herein may refer to single administration or multiple administrations (hereinafter, also referred to as "continuous administration"). Further, when comparing the scores indicating the degrees of severity of psoriasis described above before and after administration, the score of the first administration and the score after single or multiple administrations can be compared, or the scores between suitable two points in continuous administration can be compared.

A dose of the IL-17RA antagonist for use of the present invention is not particularly limited, and is desirably 70 mg or more, more desirably 140 mg or more, most desirably 210 mg or more. The dose can be suitably increased or decreased during the continuous administration of the therapeutic agent or the antagonist.

The dosing interval of the therapeutic agent and the antagonist is not particularly limited. Specifically, for example, the therapeutic agent and the antagonist may be administered on the 1^{st} day (hereinafter, also referred to as week 0), the 1^{st} week and the 2 ^{nd}week, and thereafter once every two weeks or once every four weeks. The dosing interval can also be appropriately extended, or can be shortened.

The dosing period of the therapeutic agent and the antagonist is not particularly limited. Specifically, for example, the therapeutic agent and the antagonist can be administered for 10, 20, 30, 40, or 50 weeks, or longer than 50 weeks after the first administration, and is preferably administered for longer than 50 weeks. The dosing period can include a rest period.

The IL-17RA in the present invention is not limited to a particular species, and is preferably human IL-17RA. IL-17RA antagonists inhibit the interaction between IL-17RA and a IL-17RA ligand (such as IL-17A, IL-17F, IL-17A/F, IL-25, or the like). Specifically, for example, an IL-17RA antagonist can be an antagonist having the activity to inhibit the binding of IL-17RA and the ligand, an antagonist having the activity to inhibit the IL-17RA activation initiated by the binding of the ligand, or an antagonist having the activity to inhibit the association between IL-17RA and a receptor forming a heterodimer.

The IL-17RA antagonist can have any form, including, for example, a small molecule, an antibody, an antibody fragment, an antigen-binding fragment, siRNA, an anti-sense oligomer, and the like. Preferably, the antagonist is an antibody, or an antibody fragment.

Specific examples of antibodies as IL-17RA antagonists include anti-IL-17RA antibody, anti-IL-17A antibody, anti-IL-17F antibody, anti-IL-17A/F antibody, and anti-IL-17E antibody. Preferred is anti-IL-17RA antibody.

The antibody used in the present invention can be any one of monoclonal antibody and polyclonal antibody, and preferably a monoclonal antibody which binds to a single epitope.

The antibody can be a monoclonal antibody produced from hybridomas or a genetically recombinant antibody produced by a gene recombination technique. Examples of the genetically recombinant antibody include a mouse antibody, a rat antibody, a human chimeric antibody (hereinafter, simply referred to as chimeric antibody), a humanized antibody [also called human complementarity determining region (CDR)-grafted antibody], and a human antibody; and in order to reduce immunogenicity in humans, a chimeric antibody, a humanized antibody, or a human antibody is preferably used.

Specifically, the monoclonal antibody of the present invention is an antibody selected from the following (A) to (B):
(A) a monoclonal antibody in which CDR1, CDR2 and CDR3 of a heavy chain variable region (hereinafter, referred to as VH) of the antibody comprise the amino acid sequences shown in SEQ ID NOs:1, 2 and 3, respectively, and CDR1, CDR2 and CDR3 of a light chain variable region (hereinafter, referred to as VL) of the antibody comprise the amino acid sequences shown in SEQ ID NOs:4, 5 and 6, respectively; and
(B) a monoclonal antibody in which VH of the antibody comprises the amino acid sequence shown in SEQ ID NO:7, and VL of the antibody comprises the amino acid sequence shown in SEQ ID NO:8.

In the present invention, the CDR1, CDR2 and CDR3 of VH of the antibody comprise the amino acid sequences shown in SEQ ID NOs:1, 2 and 3, respectively, and CDR1, CDR2 and CDR3 of VL of the antibody comprise the amino acid sequences shown in SEQ ID NOs:4, 5 and 6, respectively. In some embodiments, the monoclonal antibody is a monoclonal antibody in which VH of the antibody comprises the amino acid sequence shown in SEQ ID NO:7, and VL of the antibody comprises the amino acid sequence shown in SEQ ID NO:8, which includes anti-human IL-17RA human monoclonal antibody AM_{H}14/AM_{L}14 (WO 2008/054603), and genetically recombinant anti-human IL-17RA human antibody AM-14 (also referred to as brodalumab), and the like.

In the present invention, the monoclonal antibody refers to an antibody that is secreted by an antibody-producing cell of a single clone, and recognizes only one epitope (antigenic determinant), and the amino acid sequence (primary structure) constituting the monoclonal antibody is uniform.

The epitope refers to a single amino acid sequence, a three-dimensional structure consisting of the amino acid sequence, an amino acid sequence bound with a modified residue such as a sugar chain, glycolipid, polysaccharide lipid, an amino group, a carboxyl group, phosphoric acid, sulfuric acid, and the like, and a three-dimensional structure consisting of said modified residue-bound amino acid sequence, which the monoclonal antibody recognizes for binding. The three-dimensional structure is a three-dimensional structure of naturally occurring proteins, which refers to a three-dimensional structure constituted with proteins that are expressed intracellularly or on a cell membrane.

In the present invention, the antibody molecule is also called immunoglobulin (hereinafter, referred to as "Ig"). Human antibodies are classified into isotypes of IgA1, IgA2, IgD, IgE, IgG1, IgG2, IgG3, IgG4 and IgM according to the difference between molecular structures. IgG1, IgG2, IgG3, and IgG4 which are relatively highly homologous to each other in terms of the amino acid sequence are also collectively called IgG.

In the present invention, the antibody molecule consists of polypeptides called a heavy chain (hereinafter, referred to as H-chain) and a light chain (hereinafter, referred to as L-chain).

Further, the H chain consists of VH and an H-chain constant region (also referred to as CH) from the N-terminal, and the L chain consists of VL and an L-chain constant region (also referred to as CL) from the N-terminal, respectively.

A chimeric antibody refers to an antibody which consists of VH and VL of an antibody derived from a non-human animal and CH and CL of a human antibody. The species of animal from which the variable region is derived is not particularly limited, as long as it is an animal that can be used for the preparation of hybridomas, such as mouse, rat, hamster, rabbit or the like.

A humanized antibody is an antibody which is obtained by grafting CDRs of VH and VL derived from a non-human animal antibody into appropriate positions of VH and VL of a human antibody (human CDR-grafted antibody).

A human antibody means an antibody naturally existing in the human body, and also includes an antibody obtained from a human antibody phage library or a human antibody-producing transgenic animal, which is prepared based on the recent advanced techniques in genetic engineering, cell engineering and developmental engineering.

In the present invention, the type of the antibody fragment is not particularly limited, and examples thereof can include Fab, Fab', F(ab')₂, scFv, diabody, dsFv, a peptide containing CDR and the like.

The therapeutic agent of the present disclosure can be one which includes only the IL-17RA antagonist as an active ingredient, however, it is desirable to provide as a pharmaceutical formulation which is usually mixed together with one or more pharmacologically acceptable carriers and is manufactured by any well-known method in a technical field of pharmaceutics. Specific examples of the pharmaceutical formulation include a pharmaceutical formulation which comprises 140 mg/mL of genetically recombinant anti-human IL-17RA human antibody AM-14 as an active ingredient and which is formulated with 10 mmol/L of L-glutamic acid, 3% (w/v) of L-proline and 0.010% (w/v) of polysorbate 20 as an additive agent, and the like. The pharmaceutical formulation also includes a pharmaceutical formulation of pH 4.8 which comprises 140 mg/mL of a genetically recombinant anti-human IL-17RA human antibody AM-14 and further comprises 30 mmol/L of L-glutamic acid, 2.4% (w/v) of L-proline, and 0.01% (w/v) of polysorbate 20 as an additive agent. The pharmaceutical formulation can be produced, for example, by the method described in WO2011/088120.

As to an administration route of the therapeutic agent of the present disclosure or the IL-17RA antagonist which is administered in the method of the present disclosure, it is preferable to use one which is most effective during treatment. Specific examples of the administration route include an oral administration or a parenteral administration such as intraoral, airway, intrarectal, subcutaneous, intramuscular or intravenous administration, and a subcutaneous or intravenous administration is preferable and a subcutaneous administration is more preferable. Examples of an administration form include spray, capsule, tablet, powder, granule, syrup, emulsion, suppository, injection, ointment, tape and the like and injection is preferable.

An administration device of the therapeutic agent or the IL-17RA antagonist can be appropriately selected during treatment. Examples thereof include a prefilled syringe and an auto injector, however, the device is not limited to the examples.

### Example 1

Phase III Clinical Trial of Recombinant Anti-Human IL-17RA Human Antibody AM-14 in Japan

The outline of the phase III clinical trial (hereinafter, also referred to simply as "clinical trial") of the recombinant anti-human IL-17RA human antibody AM-14 (hereinafter, "AM-14") in Japan is presented in Table 1. Pustular psoriasis patients or psoriatic erythroderma patients that satisfy the conditions presented in Table 2 were selected as clinical trial subjects, and evaluated for the safety and efficacy or the like of AM-14 after chronic administration. Each subject was administered with 140 mg of AM-14 on the 1^{st} day, the 1^{st} week and the 2^{nd} week, and thereafter once every two weeks to the 50^{th} week. For subjects in which a sufficient effect was not obtained at the 4^{th} week, the dose was increased and 210 mg of AM-14 was administered once every two weeks from the 6^{th} week. The clinical trial ended at the 52^{nd} week after the administration.

AM-14 had an antibody variable region amino acid sequences described in WO 2008/054603, and was prepared as a recombinant human antibody derived from a CHO cell by using an ordinary method.

**[Table 1]**

| Indication, Design | Dose, Duration | No. Subjects Enrolled |
|---|---|---|
| Indication; generalized pustular psoriasis (GPP) or psoriatic erythroderma (PsE) Design; Open-label, Uncontrolled Study | Dose; 140 mg (dosage may be increased to 210 mg in case of insufficient efficacy) Duration; 50 weeks | 12 patients (GPP), 18 patients (PsE) |

**[Table 2]**

| |
|---|
| Ages Eligible for Study: 18 Years and older |
| Genders Eligible for Study: Both |
| Accepts Healthy Volunteers: No |
| Criteria |
| 1) Inclusion Criteria: |
| •Subject has signed voluntarily the written informed consent form to participate in this study. |
| •Subject has been diagnosed as pustular psoriasis or psoriatic erythroderma. |
| •Subject has received at least one previous phototherapy or systemic psoriasis therapy or has been a candidate to receive phototherapy or systemic psoriasis therapy in the opinion of the investigator. |
| |
| 2) Exclusion Criteria: |
| •Subject with psoriatic erythroderma has involved Body surface area (BSA) of lesion < 80% at baseline. |
| •Subject diagnosed with psoriasis guttata, medication-induced or medication-exacerbated psoriasis. |
| •Evidence of skin conditions at the time of the screening visit (eg, eczema) that would interfere with evaluations of the effect of AM-14 on psoriasis. |
| •Subject has any active Common Terminology Criteria for Adverse Events (CTCAE) grade 2 or higher infection |
| •Subject has a significant concurrent medical condition or laboratory abnormalities, as defined in the study protocol. |
| •Subject has used Ultra Violet B (UVB) therapy within 14 days of the first dose or Ultra Violet A (UVA) (with or without psoralen) within 28 days of the first dose. |
| •Subject has used etanercept, adalimumab, infliximab or ustekinumab within 1 week, 2 weeks, 8 weeks or 12 weeks of the first dose, respectively. |
| •Subject has stopped ustekinumab or other anti-lnterleukin (IL)-23 biologics therapy due to lack of efficacy |
| •Subject has used live vaccine within 3 months of the first dose |
| •Subject has previously used an anti-IL-17 biologic therapy |

### Example 2

Therapeutic Effect of AM-14 on Pustular Patients that was administered with an Anti-TNF-alpha Antibody

Five of the subjects participated in the clinical trial of Example 1 had been administered with the anti-TNF-alpha antibody. These subjects were measured for the clinical global impression (hereinafter, referred to as CGI) which represents the change in skin symptoms of psoriasis from the first administration of AM-14, over a time course. The obtained results are presented in Figs. 1(A) to 1(C), 2(D) and 2(E). CGI was shown in scales of 1 to 4 representing remission, improvement, unchanged, and aggravation, respectively, as determined by physician.

As shown in Figs. 1(A) to 1(C), 2(D) and 2(E), AM-14 were able to be administered for 50 weeks to four out of the five pustular patients that had been administered with the anti-TNF-alpha antibody, and the CGI at the 52^{nd} week was 2 or less. As demonstrated above, AM-14 was shown to have a therapeutic effect for pustular patients that had been administered with the anti-TNF-alpha antibody.

### Example 3

Therapeutic Effect of AM-14 on Pustular Psoriasis Patients that could not be treated with an Anti-TNF-alpha Antibody

Table 3 presents histories of psoriasis therapeutic agent use in the five subjects examined in Example 2. Subjects B and D in Table 3 used the psoriasis therapeutic agents in the orders shown. The three subjects A, C, and E had been using infliximab until just before AM-14 administration. Infliximab and adalimumab are anti-human TNF-alpha antibodies (infliximab is a chimeric antibody, and adalimumab is a humanized antibody), and ustekinumab is an anti-human IL-12/23p40 humanized antibody. These psoriasis therapeutic agents are all approved in Japan. The reason for withdrawal of these drugs is given as "primary failure" when the drug efficacy was insufficient from the start of administration, and "secondary failure" when the drug efficacy attenuated over the course of continuous administration in Table 3. A case where the drug administration needed to be discontinued because of not efficacy but severe side effects to a degree at which a subject cannot tolerate was described as insufficient tolerability in Table 3. AM-14 was determined as being therapeutically effective when the CGI at the 52^{nd} week was 2 or 1, and ineffective when the CGI score was other than 2 and 1.

**[Table 3]**

| Histories of Using Psoriasis Therapeutic Agent | | | |
|---|---|---|---|
| Subject | Psoriasis therapeutic agent administered | Reason for withdrawal of psoriasis therapeutic agent administered | Therapeutic effect of AM-14 |
| A | Infliximab | Primary failure | Present |
| B | Infliximab | Secondary failure | Present |
| | Adalimumab | Secondary failure | |
| | Ustekinumab | Started a new treatment | |
| C | Infliximab | Secondary failure | Present |
| D | Infliximab | Insufficient tolerability | Present |
| | U stekinumab | Stopped hospital visit | |
| | Adalimumab | Insufficient tolerability | |
| E | Infliximab | Secondary failure | Absent (withdrawn during trial) |

As shown in Table 3, the AM-14 administration was shown to have a therapeutic effect in three (subjects A, B, and C) of the four subjects (A, B, C, and E) that did not respond to infliximab or adalimumab (primary failure or secondary failure). As shown in the results of the subject D, it was found that AM-14 was able to be continuously administered for 50 weeks and shown therapeutic effect even for subjects that suffered from severe side effects by administration of infliximab and adalimumab and were determined to be insufficient tolerability to infliximab and adalimumab.

As demonstrated by these results, it was shown that, for a subject having pustular psoriasis that could not be treated with the anti-TNF-alpha antibody because the anti-TNF-alpha antibody such as infliximab and adalimumab does not work, or causes severe side effects, AM-14 is able to be continuously administered over a long period of time and to improve psoriasis symptoms.

### Example 4

Therapeutic Effect of AM-14 on Psoriatic Erythroderma patients that have been administered with an Anti-TNF-alpha Antibody

Three of the psoriatic erythroderma patients participated in the clinical trial of Example 1 had a history of anti-TNF-alpha antibody administration. These subjects were measured for PASI (Psoriasis Area and Severity Index) score, a measure of the degree of severity of psoriasis, over a time course. The results are presented in Fig. 3. PASI score was calculated by using the evaluation method given in Table 4.

**[Table 4]**

| PASI Score Calculation Method | | | | | |
|---|---|---|---|---|---|
| | | Head | Trunk | Arms | Legs |
| Observation of skin | Erythema (a) | The skin condition of each subject was scored in 4 different parts of the body (head, trunk, Arms, and Legs) according to the following criteria. Absent: 0; mild: 1; moderate: 2; severe: 3; very severe: 4 | | | |
| | Infiltration (b) | | | | |
| | Desquamation (c) | | | | |
| d = sum of a + b + c | | | | | |
| Focus area (e) | | The percentage area (%) of lesion in body surface area was given focus area points, as follows: | | | |
| | | 0%: 0; | | | |
| | | less than 10%: 1; | | | |
| | | 10% or more and less than 30%: 2; | | | |
| | | 30% or more and less than 50%: 3; | | | |
| | | 50% or more and less than 70%: 4; | | | |
| | | 70% or more and less than 90%: 5; and | | | |
| | | 90 to 100%: 6. | | | |
| d × e × coefficient by body part | | d × e × 0.1 (I) | d × e × 0.3 (II) | d × e × 0.2 (III) | d × e × 0.4 (IV) |
| PASI score | | Sum of (I) + (II) + (III) + (IV) | | | |

As described in Fig. 3, the PASI score decreased in each subject as the number of administration increased. The PASI score decreased to nearly 0 at the 10^{th}, the 12^{th}, and the 48^{th} week from the first administration in subjects F, H, and G, respectively. As demonstrated by these results, AM-14 was shown to have therapeutic effect for psoriatic erythroderma patients that had been administered with the anti-TNF-alpha antibodies.

Table 5 presents the histories of psoriasis therapeutic agent use for these three subjects.

**[Table 5]**

| Subjects' Histories of Using Psoriasis Therapeutic Agent | | | |
|---|---|---|---|
| Subject | Psoriasis therapeutic agent administered | Reason for withdrawal of psoriasis therapeutic agent administered | AM-14 therapeutic effect |
| F | Adalimumab | Other (Change of treatment plan) | Present |
| G | Infliximab | Insufficient tolerability | Present |
| H | Adalimumab | Primary failure | Present |
| | Ustekinumab | Other (convulsive seizure) | |

Table 5 follows the same definitions used in Table 3. AM-14 was determined as being therapeutically effective when the PASI score at the 52^{nd} week was lower than that measured before the administration of AM-14 in each patient. As can be seen in Table 5, AM-14 was shown to be therapeutically effective also in the patient (subject H) that did not respond to adalimumab. Although severe side effects occurred by administration of infliximab in the subject (subject G), and the subject G was a subject that was determined to be insufficient tolerability to infliximab, AM-14 was able to be continuously administered for 50 weeks, and shown therapeutic effect.

As demonstrated by these results, it was shown that, for a subject of psoriatic erythroderma that could not be treated with the anti-TNF-alpha antibody because the anti-TNF-alpha antibody such as infliximab and adalimumab does not work, or causes severe side effects, AM-14 is able to be continuously administered over a long period of time and to improve psoriasis symptoms.

### Sequence Listing Free Text

SEQ ID NO: 1 - Description of artificial sequence: amino acid sequence of AM-14_HCDR1
SEQ ID NO: 2 - Description of artificial sequence: amino acid sequence of AM-14_HCDR2
SEQ ID NO: 3 - Description of artificial sequence: amino acid sequence of AM-14_HCDR3
SEQ ID NO: 4 - Description of artificial sequence: amino acid sequence of AM-14_LCDR1
SEQ ID NO: 5 - Description of artificial sequence: amino acid sequence of AM-14_LCDR2
SEQ ID NO: 6 - Description of artificial sequence: amino acid sequence of AM-14_LCDR3
SEQ ID NO: 7 - Description of artificial sequence: amino acid sequence of AM-14_VH
SEQ ID NO: 8 - Description of artificial sequence: amino acid sequence of AM-14_VL

## Claims

1. An IL-17RA antagonist for use in a method for the treatment of psoriasis, wherein the method comprises administering an IL-17RA antagonist to a psoriasis patient that cannot be treated with an anti-TNF-alpha antibody,
wherein the patient that cannot be treated with the anti-TNF-alpha antibody is:
(a) a patient that is ineffective to the anti-TNF-alpha antibody, wherein ineffective means a clinical global impression (CGI) of 3 or 4, or
(b) a patient that is of insufficient tolerability to the anti-TNF-alpha antibody, wherein insufficient tolerability means severe side effects to a degree at which a patient cannot tolerate occur,
wherein the IL-17RA antagonist is an anti-IL-17RA antibody or an antibody fragment thereof, and wherein the anti-IL-17RA antibody is a monoclonal antibody in which CDR1, CDR2, and CDR3 of a heavy chain variable domain (VH) of the antibody comprise the amino acid sequences shown in SEQ ID NOs:1, 2, and 3, respectively, and CDR1, CDR2, and CDR3 of a light chain variable domain (VL) of the antibody comprise the amino acid sequences shown in SEQ ID NOs:4, 5, and 6, respectively,
and wherein the anti-TNF-alpha antibody is at least one selected from adalimumab, infliximab, certolizumab pegol, certolizumab, and golimumab.

2. The IL-17RA antagonist for use according to claim 1, wherein the psoriasis is at least one selected from psoriasis vulgaris, psoriasis arthropica, pustular psoriasis, psoriatic erythroderma, and psoriasis guttata.

3. The IL-17RA antagonist for use according to claim 1 or claim 2, wherein the anti-IL-17RA antibody is a monoclonal antibody in which VH of the antibody comprises the amino acid sequence shown in SEQ ID NO:7, and VL of the antibody comprises the amino acid sequence shown in SEQ ID NO:8.

4. The IL-17RA antagonist for use according to claim 3, wherein the anti-IL-17RA antibody is a human IgG2 antibody.

5. The IL-17RA antagonist for use according to claim 4, wherein the anti-IL-17RA antibody is a genetically recombinant antibody that is formulated in a pharmaceutical formulation of pH 4.8 which comprises 140 mg/mL of the antibody, 30 mmol/L of L-glutamic acid, 2.4% (w/v) of L-proline, and 0.01% (w/v) of polysorbate 20 as an additive agent

6. The IL-17RA antagonist for use according to any one of claims 1-5, wherein the CGI of the patient after the administration of the IL-17RA antagonist becomes 2 or 1.

7. The IL-17RA antagonist for use according to any one of claims 1-6, wherein the psoriasis area and severity index (PASI) score of a patient after the administration of the IL-17RA antagonist is lower than that before the administration of the antagonist.

8. The IL-17RA antagonist for use according to any one of claims 1-7, wherein the IL-17RA antagonist is administered in a dose of 140 mg or more.

9. The IL-17RA antagonist for use according to any one of claims 1-8, wherein the IL-17RA antagonist is administered in a dose of 140 mg or 210 mg.

10. The IL-17RA antagonist for use according to any one of claims 1-9, wherein the IL-17RA antagonist is administered in a dose of 140 mg or 210 mg on the 1^{st} day, the 1^{st} week and the 2^{nd} week, and thereafter once every two weeks.

## Patentansprüche

1. IL-17RA-Antagonist zur Verwendung in einem Verfahren zur Behandlung von Psoriasis, wobei das Verfahren die Verabreichung eines IL-17RA-Antagonisten an einen Psoriasispatienten, der nicht mit einem Anti-TNF-alpha-Antikörper behandelt werden kann, umfasst,
wobei es sich bei dem Patienten, der nicht mit dem Anti-TNF-alpha-Antikörper behandelt werden kann, um:
(a) einen Patienten, bei dem der Anti-TNF-alpha-Antikörper unwirksam ist, wobei unwirksam eine Clinical Global Impression (CGI) von 3 oder 4 bedeutet, oder
(b) einen Patienten, der den Anti-TNF-alpha-Antikörper unzureichend toleriert, wobei unzureichend toleriert schwere Nebenwirkungen bis zu einem Grad, den ein Patient nicht tolerieren kann, bedeutet,
handelt, wobei es sich bei dem IL-17RA-Antagonisten um einen Anti-IL-17RA-Antikörper oder ein Antikörperfragment davon handelt und wobei es sich bei dem Anti-IL-17RA-Antikörper um einen monoklonalen Antikörper handelt, bei dem CDR1, CDR2 und CDR3 einer variablen Domäne der schweren Kette (VH) des Antikörpers die in SEQ ID NO: 1, 2 bzw. 3 gezeigten Aminosäuresequenzen umfassen und CDR1, CDR2 und CDR3 einer variablen Domäne der leichten Kette (VL) des Antikörpers die in SEQ ID NO: 4, 5 bzw. 6 gezeigten Aminosäuresequenzen umfassen,
und wobei es sich bei dem Anti-TNF-alpha-Antikörper um Adalimumab, Infliximab, Certolizumab pegol, Certolizumab und/oder Golimumab handelt.

2. IL-17RA-Antagonist zur Verwendung nach Anspruch 1, wobei es sich bei der Psoriasis um Psoriasis vulgaris, Psoriasis arthropica, Pustelpsoriasis, psoriatische Erythrodermie und/oder Psoriasis guttata handelt.

3. IL-17RA-Antagonist zur Verwendung nach Anspruch 1 oder Anspruch 2, wobei es sich bei dem Anti-IL-17RA-Antikörper um einen monoklonalen Antikörper handelt, bei dem die VH des Antikörpers die in SEQ ID NO: 7 gezeigte Aminosäuresequenz umfasst und die VL des Antikörpers die in SEQ ID NO: 8 gezeigte Aminosäuresequenz umfasst.

4. IL-17RA-Antagonist zur Verwendung nach Anspruch 3, wobei es sich bei dem Anti-IL-17RA-Antikörper um einen humanen IgG2-Antikörper handelt.

5. IL-17RA-Antagonist zur Verwendung nach Anspruch 4, wobei es sich bei dem Anti-IL-17RA-Antikörper um einen genetisch rekombinanten Antikörper handelt, der in einer pharmazeutischen Formulierung mit einem pH-Wert von 4,8 formuliert ist, die 140 mg/ml des Antikörpers, 30 mmol-1 L-Glutaminsäure, 2,4% (w/v) L-Prolin und 0,01% (w/v) Polysorbat 20 als Additiv umfasst.

6. IL-17RA-Antagonist zur Verwendung nach einem der Ansprüche 1-5, wobei die CGI des Patienten nach der Verabreichung des IL-17RA-Antagonisten 2 oder 1 wird.

7. IL-17RA-Antagonist zur Verwendung nach einem der Ansprüche 1-6, wobei die Psoriasis Area and Severity Index(PASI)-Bewertung eines Patienten nach der Verabreichung des IL-17RA-Antagonisten niedriger ist als vor der Verabreichung des Antagonisten.

8. IL-17RA-Antagonist zur Verwendung nach einem der Ansprüche 1-7, wobei der IL-17RA-Antagonist in einer Dosis von 140 mg oder mehr verabreicht wird.

9. IL-17RA-Antagonist zur Verwendung nach einem der Ansprüche 1-8, wobei der IL-17RA-Antagonist in einer Dosis von 140 mg oder 210 mg verabreicht wird.

10. IL-17RA-Antagonist zur Verwendung nach einem der Ansprüche 1-9, wobei der IL-17RA-Antagonist am ersten Tag der 1. Woche und der 2. Woche und anschließend einmal alle zwei Wochen in einer Dosis von 140 mg oder 210 mg verabreicht wird.

## Revendications

1. Antagoniste de l'IL-17RA destiné à être utilisé dans un procédé destiné au traitement du psoriasis, dans lequel le procédé comprend une administration d'un antagoniste de l'IL-17RA à un patient atteint de psoriasis qui ne peut pas être traité avec un anticorps anti-TNF-alpha,
dans lequel le patient qui ne peut pas être traité avec l'anticorps anti-TNF-alpha est :
(a) un patient pour qui l'anticorps anti-TNF-alpha est inefficace, dans lequel inefficace signifie une impression clinique globale (CGI) de 3 ou 4, ou
(b) un patient qui a une tolérance insuffisante à l'anticorps anti-TNF-alpha, dans lequel une tolérance insuffisante signifie des effets secondaires ayant un degré de gravité tel qu'un patient ne puisse pas supporter qu'ils apparaissent,
dans lequel l'antagoniste de l'IL-17RA est un anticorps anti-IL-17RA ou un fragment d'anticorps de celui-ci, et dans lequel l'anticorps anti-IL-17RA est un anticorps monoclonal dans lequel des CDR1, CDR2 et CDR3 d'un domaine variable de chaîne lourde (VH) de l'anticorps comprennent les séquences d'acides aminés présentées dans les SEQ ID n° : 1, 2 et 3 respectivement, et des CDR1, CDR2 et CDR3 d'un domaine variable de chaîne légère (VL) de l'anticorps comprennent les séquences d'acides aminés présentées dans les SEQ ID n° : 4, 5 et 6 respectivement,
et dans lequel l'anticorps anti-TNF-alpha en est au moins un choisi parmi les adalimumab, infliximab, certolizumab pegol, certolizumab et golimumab.

2. Antagoniste de l'IL-17RA destiné à être utilisé selon la revendication 1, dans lequel le psoriasis en est un choisi parmi les psoriasis vulgaire, psoriasis arthropathique, psoriasis pustuleux, psoriasis érythrodermique et psoriasis en gouttes.

3. Antagoniste de l'IL-17RA destiné à être utilisé selon la revendication 1 ou la revendication 2, dans lequel l'anticorps anti-IL-17RA est un anticorps monoclonal dans lequel le VH de l'anticorps comprend la séquence d'acides aminés présentée dans la SEQ ID n° : 7 et le VL de l'anticorps comprend la séquence d'acides aminés présentée dans la SEQ ID n° : 8.

4. Antagoniste de l'IL-17RA destiné à être utilisé selon la revendication 3, dans lequel l'anticorps anti-IL-17RA est un anticorps IgG2 humain.

5. Antagoniste de l'IL-17RA destiné à être utilisé selon la revendication 4, dans lequel l'anticorps anti-IL-17RA est un anticorps recombinant par génie génétique qui est formulé dans une formulation pharmaceutique de pH 4,8, qui comprend l'anticorps à 140 mg/mL, de l'acide L-glutamique à 30 mmol/L, de la L-proline à 2,4% (p/v) et du polysorbate 20 à 0,01% (p/v) comme agent additif

6. Antagoniste de l'IL-17RA destiné à être utilisé selon l'une quelconque des revendications 1 à 5, dans lequel la CGI du patient après l'administration de l'antagoniste de l'IL-17RA devient 2 ou 1.

7. Antagoniste de l'IL-17RA destiné à être utilisé selon l'une quelconque des revendications 1 à 6, dans lequel le score de l'indice de gravité et d'étendue du psoriasis (PASI) d'un patient après l'administration de l'antagoniste de l'IL-17RA est inférieur à celui d'avant l'administration de l'antagoniste.

8. Antagoniste de l'IL-17RA destiné à être utilisé selon l'une quelconque des revendications 1 à 7, dans lequel l'antagoniste de l'IL-17RA est administré en une dose de 140 mg ou plus.

9. Antagoniste de l'IL-17RA destiné à être utilisé selon l'une quelconque des revendications 1 à 8, dans lequel l'antagoniste de l'IL-17RA est administré en une dose de 140 mg ou de 210 mg.

10. Antagoniste de l'IL-17RA destiné à être utilisé selon l'une quelconque des revendications 1 à 9, dans lequel l'antagoniste de l'IL-17RA est administré en une dose de 140 mg ou de 210 mg le 1^{er} jour, la 1^{ère} semaine et la 2^{ème} semaine et ensuite une fois toutes les deux semaines.
